# EUROPEAN PATENT APPLICATION

(11) **EP 4 566 630 A1**
(43) Date of publication of application: **11.06.2025**
(21) Application number: 23850070.6
(22) Date of filing: 01.08.2023
(51) Int. Cl.: A61K 47/36, A61K 9/14, A61K 45/00

(54) **POROUS CARRIER PARTICLES, FUNCTIONAL COMPONENT-CARRIED PARTICLES, AND METHOD FOR PRODUCING POROUS CARRIER PARTICLES**

(30) Priority: 01.08.2022 JP 2022122676
(71) Applicant: TOWA PHARMACEUTICAL CO., LTD., Kadoma-shi, Osaka 571-8580 (JP)
(72) Inventor: OKUSHIMA Tomoaki, Kadoma-shi, Osaka 571-8580 (JP); OKUDA Yutaka, Kadoma-shi, Osaka 571-8580 (JP)
(74) Representative: V.O.
(86) International application number: PCT/JP2023/028068
(87) International publication number: WO 2024/029516

(57) **Abstract**

Porous carrier particles include a water-soluble polymer that has a helical structure using glucose as a unit, in which the porous carrier particles have a BET specific surface area of 1 m²/g or greater. It is preferable that the water-soluble polymer is a water-soluble polysaccharide. It is preferable that the water-soluble polysaccharide is one or more selected from the group consisting of dextrin, dextran, agarose, and pullulan.

## Description

### TECHNICAL FIELD

The present invention relates to porous carrier particles, particles carrying functional ingredient and a method of producing porous carrier particles.

Priority is claimed on Japanese Patent Application No. 2022-122676, filed August 1, 2022, the content of which is incorporated herein by reference.

### BACKGROUND ART

A method of administering a functional ingredient such as an active pharmaceutical ingredient to a body includes local administration, enteral administration, and parenteral administration. The administered functional ingredient needs to be transported from a site administered to a target site where the function isexpressed.

In a case of oral administration, which is one of the enteral administration, the functional ingredient passes through the mouth and the stomach and is absorbed in the small intestine. Therefore, the functional ingredient needs to be transported to the small intestine.

In order to transport a functional ingredient to a target site in the body, a mechanism of carrying a functional ingredient inside the particle called a carrier is useful. The carrier is required to exhibit a large capacity of a functional ingredient, for example, with a small particle diameter. As such a material, a porous inorganic material has been examined.

For example, Patent Document 1 discloses a crystalline mesoporous silica material having a skeleton of a zeolite-type micropore (clearly referred to as a structural unit having a size of nanometers) and not causing Bragg diffraction in X-ray diffraction, and use of the material that delivers a drug. Patent Document 1 discloses that the release rate of a drug is less than 80% in a case where a zeolite material is used.

### Citation List

### Patent Document

Patent Document 1: Published Japanese Translation No. 2007-523817 of the PCT International Publication

### SUMMARY OF INVENTION

### Technical Problem

A porous material formed of an inorganic compound is likely to increase the specific surface area.

Meanwhile, an inorganic compound is water-insoluble and cannot be dissolved in a body. In this case, there is a problem in that all the functional ingredients carried by a carrier are not released, and a desired dose cannot be administered to the body.

The present invention has been made in consideration of the above-described circumstances, and an object thereof is to provide water-soluble porous carrier particles which are capable of carrying a functional ingredient and have a high BET specific surface area.

### Solution to Problem

The present invention includes the following aspects [1] to [7].
[1] Porous carrier particles: a water-soluble polymer that has a helical structure including glucose as a unit, in which the porous carrier particles have a BET specific surface area of 1 m²/g or greater.
[2] The porous carrier particles according to [1], in which the water-soluble polymer is a water-soluble polysaccharide.
[3] The porous carrier particles according to [1] or [2], in which the water-soluble polysaccharide is one or more selected from the group consisting of dextrin, dextran, agarose, and pullulan.
[4] The porous carrier particles according to any one of [1] to [3], in which the porous carrier particles are spherical.
[5]Particles carrying functional ingredient, in which a functional ingredient is retained inside the porous carrier particles according to any one of [1] to [4].
[6] The particles carrying functional ingredient according to [5], in which the functional ingredient is an active pharmaceutical ingredient.
[7] A method of producing water-soluble porous carrier particles having a BET specific surface area of 1 m²/g or greater, the method including: a step of spray-drying a solution that contains a water-soluble polymer having a helical structure including glucose as a unit to obtain precursor particles; and a step of mixing an organic solvent with the precursor particles to obtain a mixture and removing a liquid from the mixture. Advantageous Effects of Invention

According to the present invention, it is possible to provide water-soluble porous carrier particles which are capable of carrying a functional ingredient and have a high BET specific surface area.

### BRIEF DESCRIPTION OF DRAWINGS

[FIG. 1] An electron micrograph showing porous carrier particles produced in Example 1.
[FIG. 2] An electron micrograph showing porous carrier particles produced in Example 2.

### DESCRIPTION OF EMBODIMENTS

### <Porous carrier particles>

Porous carrier particles of the present invention contain a water-soluble polymer and are water-soluble. Hereinafter, the porous carrier particles of the present invention will also be referred to as "carrier particles". The water-soluble carrier particles are completely dissolved in vivo. Therefore, both the carried functional ingredient and the carrier particles are completely dissolved in vivo, and the functional ingredient can be completely released. In addition, since the carrier particles are porous, the contact area with the surrounding medium can be increased, and thus quick solubility can also be exhibited.

As the water-soluble porous particles, for example, the known water-soluble porous particles described in Japanese Unexamined Patent Application, First Publication No. H4-335870 have an extremely small BET specific surface area of 0.2 m²/g or less.

On the contrary, the carrier particles of the present invention have a specific surface area higher than water-soluble porous particles of the related art. Therefore, the amount of the functional ingredient to be carried can be increased as compared with the water-soluble porous particles of the related art.

The term "water-soluble" denotes a property of having a solubility of 1 g or greater in 100 g of distilled water at 25°C and 1 atm.

The term "porous" denotes a state where a substance has a plurality of micropores and has a BET specific surface area of 1 m²/g or greater.

It is preferable that the carrier particles are substantially formed of a water-soluble polymer.

Here, the term "substantially composed of water-soluble polymers" refers to a form that is solely composed of water-soluble polymers, or a form that includes a certain amount of other components without impairing the function of the carrier to retain the functional ingredient.

The phrase "without impairing the function of the carrier." means that the quality to retain functional ingredients is not deteriorated, and the carrier could be still safely administered in vivo as a pharmaceutical product. Specifically, the expression denotes that the content proportion of other ingredients is 1% by mass or less with respect to the entire carrier particles and preferably 0.1% by mass or less.

Hereinafter, each ingredient will be described.

### <<Water-soluble polymer>>

The water-soluble polymer in the present embodiment has a helical structure including glucose as a unit.

Whether or not the carrier particles contain a water-soluble polymer having a helical structure including glucose as a unit can be confirmed by the following method.

According to the confirmation method for dextrin described in p. 1150 in the 18th edition of the Japanese Pharmacopoeia, 100 mL of water is added to 0.1 g of the carrier particles, and one drop of an iodine test solution is added thereto. In a case where the solution exhibits a light reddish-brown color or a light reddish-violet color after the iodine test solution is added, it is determined that the carrier particles contain a water-soluble polymer having a helical structure including glucose as a unit.

The water-soluble polymer used in the present embodiment is preferably a natural polymer from the viewpoint of safely taking the carrier particles in a case of using the carrier particles for a pharmaceutical application. As the natural polymer, specifically, a water-soluble polysaccharide can be used.

The water-soluble polysaccharide used in the present embodiment is a water-soluble polymer having a helical structure, in which glucose is polymerized in a chain shape. It is preferable that the water-soluble polysaccharide has a multi-branched three-dimensional network structure.

The water-soluble polysaccharide having a three-dimensional network structure is preferably one or more selected from the group consisting of dextrin, dextran, agarose, and pullulan and more preferably dextrin.

In addition, from the viewpoint of safely taking the carrier particles, it is preferable that the water-soluble polymer does not contain a metal element. In addition, it is preferable that the entire carrier particles do not contain a metal element.

Although the details of a method of producing the carrier particles will be described below, a solution containing a water-soluble polymer is spray-dried to obtain precursor particles, and the obtained precursor particles are mixed with an organic solvent to obtain a mixture. The carrier particles are obtained by removing the liquid from the obtained mixture.

In a case where the water-soluble polymer has a three-dimensional network structure, a part of the glucose chain (for example, maltose or maltotriose) contained in the three-dimensional network structure (the precursor particles) is extracted and removed with an organic solvent, a portion where the extracted substance is present is formed into micropores, and the carrier particles are formed.

### <<Optional ingredients>>

It is preferable that the carrier particles do not contain the organic acid described below, but the carrier particles may contain a trace amount of the organic acid.

The expression "the carrier particles do not contain an organic acid" denotes that in a case where the amount of the organic acid contained in the carrier particles is measured, the measured amount of the organic acid is the detection limit or less.

In a step of producing the carrier particles, the organic acid may or may not be used, but in a case where the organic acid is used, the organic acid is finally removed. Depending on the removal method, a trace amount of the organic acid may remain in the carrier particles. In this case, it is preferable that the amount of the organic acid in the carrier particles, which is measured by the following method, is 1% or less.

### [Method of measuring organic acid in carrier particles]

The organic acid can be detected by dissolving the carrier particles in a phosphoric acid aqueous solution and analyzing the solution by high performance liquid chromatography (HPLC).

### <<BET specific surface area>>

The carrier particles have a BET specific surface area of 1 m²/g or greater, preferably 40 m²/g or greater, more preferably 50 m²/g or greater, particularly preferably 100 m²/g or greater, and still more preferably 150 m²/g or greater. The upper limit of the BET specific surface area thereof is, for example, 1000 m²/g or less, 900 m²/g or less, and 800 m²/g or less.

The upper limits and the lower limits of the BET specific surface area can be randomly combined together. Examples of the combination include 1 m²/g or greater and 1000 m²/g or less, 40 m²/g or greater and 1000 m²/g or less, 50 m²/g or greater and 1000 m²/g or less, 100 m²/g or greater and 900 m²/g or less, and 150 m²/g or greater and 800 m²/g or less.

### [Method of measuring BET specific surface area]

The BET specific surface area of the carrier particles is measured by nitrogen adsorption Brunauer, Emmett, Teller (BET) method using a specific surface area measuring device. As the specific surface area measuring device, for example, a specific surface area measuring device (device name; Macsorb, manufactured by MOUNTECH Co., Ltd.) can be used.

### <<Shape>>

The shape of the carrier particles is not particularly limited, but is preferably spherical.

The particle shape can be confirmed by static image analysis.

The static image analysis can be performed by a static automatic image analysis device. As the static automatic image analysis device, for example, Morphologi 4 (manufactured by Malvern Panalytical Ltd.) can be used.

### <<Particle diameter>>

The average particle diameter of the carrier particles can be set to be, for example, in a range of 5 µm or greater and 1000 µm or less, 10 µm or greater and 800 µm or less, 20 µm or greater and 600 µm or less, 30 µm or greater and 500 µm or less, 40 µm or greater and 400 µm or less, or 50 µm or greater and 300 µm or less.

### [Method of measuring average particle diameter]

The average particle diameter of the carrier particles can be measured by laser diffraction or laser scattering.

Hereinafter, the method of producing the carrier particles of the present invention will be described.

### <Method of producing carrier particles>

The method of producing the carrier particles will be described below by using a case of producing spherical carrier particles as an example.

Examples of the method of producing the carrier particles include the following production method 1 and production method 2.

### <<Production method 1>>

The method 1 of producing the carrier particles includes a step of obtaining precursor particles using a water-soluble polymer (production raw material) and a step of obtaining carrier particles.

### [Step of obtaining precursor particles]

First, a water-soluble polymer as a production raw material is dissolved in water to obtain an aqueous solution. Thereafter, the obtained aqueous solution is spray-dried to obtain precursor particles. A known spray-drying device can be used for the spray-drying.

The concentration of the solid content (solute) contained in the aqueous solution is preferably in a range of 30% or greater and 80% or less and more preferably 40% or greater and 60% or less.

In a case where the concentration of the solid content in the aqueous solution is equal to or greater than the above-described lower limits, the precursor particles having a solid structure filled with the content are obtained, and in a case where the precursor particles are mixed with an organic solvent in a subsequent step and the liquid is removed, more micropores are formed. In a case where the concentration of the solid content of the aqueous solution is equal to or less than the above-described upper limits, spray-drying is easily performed, and the shapes of the precursor particles and the carrier particles obtained in the subsequent step are close to a spherical shape.

As an example, the spray-drying is performed under conditions of an inlet temperature of 120°C to 160°C, an outlet temperature of 80°C to 120°C, and an aqueous solution supply rate of 5 kg/hour to 25 kg/hour.

The water-soluble polymer is the same as the description in <Porous carrier particles> above.

### [Step of obtaining carrier particles]

The precursor particles and the organic solvent are mixed, and the liquid is removed from the mixture.

As the organic solvent, a known organic solvent can be used. In the present embodiment, a hydrophilic organic solvent is preferable, and for example, ethanol or methanol is preferable as the hydrophilic organic solvent.

The precursor particles can be mixed with the organic solvent by suspending the precursor particles in the heated organic solvent and holding the organic solvent for a predetermined time or longer. The heating temperature and the heating time can be appropriately adjusted according to the organic solvent to be used, and the organic solvent is held, for example, at 0°C to 70°C for 10 minutes or longer and 120 minutes or shorter.

Subsequently, a part of the glucose chain (for example, sugar using glucose such as maltose or maltotriose as a basic unit) constituting the water-soluble polymer (production raw material) is removed from the precursor particles by removing the liquid from the mixture, and the removal trace is formed into micropores so that the carrier particles are obtained.

The liquid to be removed here is an organic solvent containing a part of the glucose chain constituting the water-soluble polymer (production raw material).

In the production method 1, examples of the water-soluble polymer used as the production raw material include dextrin.

The saccharification rate of starch of the dextrin as the production raw material is not particularly limited, and the dextrin may be dextrin having a DE value of 10 or less, maltodextrin having a DE value of greater than 10 and 20 or less, or dextrin having a DE value of greater than 20.

As a method for removing the liquid from the mixture, a known method can be used, and for example, a filtration method or a decantation method can be employed. In addition, the specific surface area of the carrier particles to be obtained can be increased by repeating a process of mixing the particles obtained by removing the liquid with an organic solvent again and removing the liquid from the mixture.

Among these, in a case where dextrin in which the content of maltose or maltotriose is high is used as the dextrin used as the production raw material, the removal trace of maltose or maltotriose is formed into micropores, and carrier particles having a high BET specific surface area are likely to be obtained.

### <<Production method 2>>

The method 2 of producing the carrier particles includes a step of obtaining precursor particles which are particles of a water-soluble polymer (production raw material) and a step of obtaining the carrier particles.

The difference from the above-described production method 1 is that a template (dosage form) is added to an aqueous solution in the step of obtaining the precursor particles. As the template, an organic acid, sugar, or sugar alcohol can be used.

### (Organic acid)

The organic acid is preferably citric acid, malic acid, tartaric acid, succinic acid, or adipic acid and more preferably citric acid, malic acid, or tartaric acid.

### (Sugar or sugar alcohol)

The sugar is, for example, a monosaccharide or a disaccharide.

As the monosaccharide, glucose, fructose, or galactose can be used.

As the disaccharide, for example, sucrose, lactose, maltose, trehalose, or cellobiose can be used. Among these, maltose or trehalose is preferable, and trehalose is particularly preferable as the disaccharide.

Examples of the sugar alcohol include mannitol, erythritol, and xylitol.

The production method 2 is preferably used in a case where dextrin in which the content of maltose or maltotriose is low is used as a production raw material. In the production method 2, the removal trace of the template added in place of maltose or maltotriose in the dextrin used as the production raw material is formed into micropores, and thus carrier particles having a high BET specific surface area are likely to be obtained.

### < Particles carrying functional ingredient>

The present invention relates to particles carrying functional ingredient in which a functional ingredient is retained inside the above-described carrier particles.

Examples of the functional ingredient include a active pharmaceutical ingredient, a pharmaceutical additive, a functional food ingredient, a coloring agent, and a flavoring agent.

The carrier particles of the present embodiment have quick solubility and can release all of the carried ingredients, and thus the carrier particles can be suitably used as a carrier for an active pharmaceutical ingredient.

As the active pharmaceutical ingredient to be carried, for example, one or two or more ingredients selected from antipyretic analgesic and anti-inflammatory drugs, psychotropic drugs, antianxiety drugs, antidepressants, sedatives, antispasmodics, central nervous system acting drugs, cerebral metabolism improvers, cerebral circulation improvers, antiepileptic drugs, sympathomimetics, gastrointestinal drugs, antacids, antiulcer drugs, antitussives, antiemetics, respiratory stimulants, bronchodilators, allergy drugs, dental and oral drugs, antihistamines, cardiac stimulants, antiarrhythmic drugs, diuretics, antihypertensive drugs, vasoconstrictors, coronary vasodilators, peripheral vasodilators, hyperlipidemic drugs, cholagogues, antibiotics, chemotherapeutic drugs, antidiabetic drugs, osteoporosis drugs, antirheumatic drugs, antispasmodics, hormones, alkaloid narcotics, sulfa drugs, gout treatment drugs, anticoagulants, antineoplastic agents, and tonic health supplements are used.

Examples of the functional food ingredient include vitamins such as vitamin A, vitamin D, and vitamin E, and higher unsaturated fatty acids such as docosahexaenoic acid (DHA), eicosapentaenoic acid (EPA), and cod liver oil.

It is preferable that a method of allowing the functional ingredient to be carried by the carrier particles is performed by impregnating the carrier particles with a desired functional ingredient. As such a method, for example, the method described in US10004682B2 can be used.

In addition, examples of the method of allowing the functional ingredient to be carried by the carrier particles include the following method.

First, the functional ingredient is dissolved in an organic solvent to obtain a functional ingredient solution. The organic solvent is, for example, ethanol. In this case, for the purpose of improving the solubility of the functional ingredient, a small amount of water (about 1% with respect to the total amount of the functional ingredient solution) may be added to the organic solvent.

Thereafter, the carrier particles and the functional ingredient solution are mixed.

Examples of a method of mixing the carrier particles with the functional ingredient solution include a method of adding the obtained functional ingredient solution dropwise to the carrier particles or spraying the obtained functional ingredient solution onto the carrier particles.

After a predetermined amount of the functional ingredient solution is mixed with the carrier particles, the mixture is dried under reduced pressure at a temperature of 20°C or higher and 60°C or lower, thereby obtaining carrier particles carrying the functional ingredient.

### <Base material for powdering oily substance>

The carrier particles of the present embodiment have a high affinity for an oily substance and can be suitably used as a base material for powdering an oily substance.

### <Catalyst or catalyst carrier>

The carrier particles of the present embodiment may be used as a catalyst or a catalyst carrier.

Examples of the catalyst to be carried include a catalyst containing platinum, palladium, or iridium as a main ingredient, and titanium oxide.

### Examples

Next, the present invention will be described in more detail with reference to examples.

### <Example 1>

### [Step of obtaining precursor particles]

2.0 kg of dextrin (GLUCIDEX IT 47) was dissolved in 3.0 kg of water heated to 70°C, thereby obtaining an aqueous solution 1. Further, the DE value of the dextrin used was 47.

The aqueous solution 1 was sprayed with a spray dryer under the following conditions, thereby obtaining precursor particles 1.

### (Spray conditions)

Inlet temperature: 140°C
Outlet temperature: 96°C to 100°C
Disk rotation speed: 5000 rpm
Supply rate of aqueous solution: 7 kg/hour

### [Process to obtain carrier particles]

60 ml of anhydrous ethanol was added to 3 g of the precursor particles, and the mixture was shaken and mixed in a water bath at 50°C for 10 minutes. After completion of the shaking, the mixture was allowed to stand for 2 minutes, and the supernatant was removed by decantation. 60 ml of anhydrous ethanol was added thereto again, and the mixture was shaken and decanted in the same manner as described above, and this operation was repeated 10 times in total from the beginning. After the final decantation, the mixture was dried overnight at 40°C and -0.1 MPa and allowed to pass through an 83M sieve, thereby obtaining carrier particles 1.

The carrier particles 1 conformed the criteria for water-soluble and porous defined in the present specification.

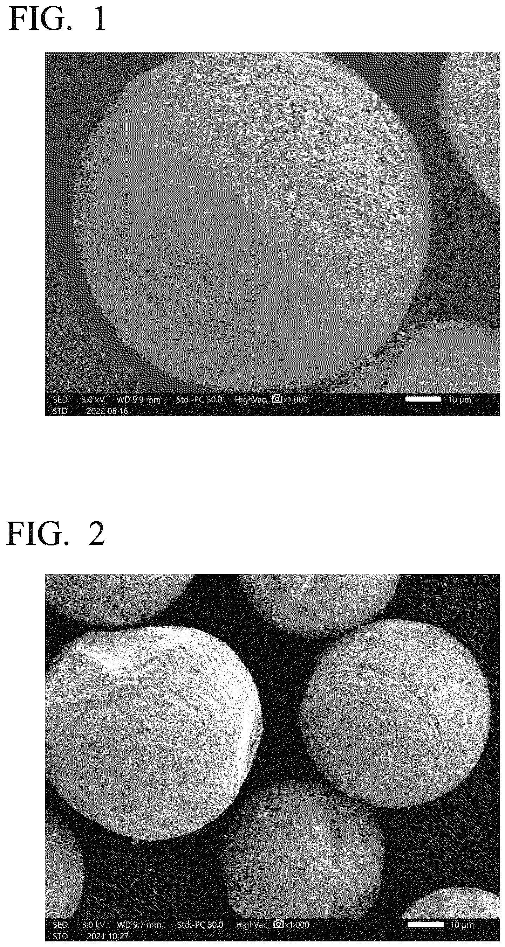

FIG. 1 shows an electron micrograph (at the magnification of 2000) of the carrier particles 1. As shown in FIG. 1, the carrier particles 1 were spherical.

The specific surface area of the carrier particles 1 measured by [Method of measuring BET specific surface area] described above was 174 m²/g.

The average pore diameter of the carrier particles 1 was 11.9 nm, and the total pore volume thereof was 0.52 cm³/g.

### <Example 2>

### [Step of obtaining precursor particles]

9 kg of water (water temperature of 70°C), 12.6 kg of trehalose (TREHALOSE P, manufactured by Hayashibara Co., Ltd.), 6.3 kg of dextrin (SANDEK #300, manufactured by Sanwa Starch Co., Ltd.), and 2.1 kg of anhydrous citric acid were mixed, thereby obtaining an aqueous solution 2 having a solid content concentration of 70%.

The DE of the dextrin used was 27.

The aqueous solution 2 obtained under the following conditions was sprayed with a spray dryer, thereby obtaining precursor particles 2.

### (Spray conditions)

Inlet temperature: 140°C
Outlet temperature: 101°C
Disk rotation speed: 8000 rpm
Supply rate of aqueous solution: 23 kg/hour

### [Step of obtaining carrier particles]

300 ml of anhydrous ethanol was added to 15 g of the obtained precursor particles, and the mixture was shaken and mixed in a water bath at 50°C for 15 minutes. After completion of the shaking, the mixture was allowed to stand for 2 minutes, and the supernatant was removed by decantation. 300 ml of anhydrous ethanol was added thereto again, and the mixture was shaken and decanted in the same manner as described above, and this operation was repeated 10 times in total from the beginning. After the final decantation, the mixture was dried at 40°C and -0.1 MPa for 2 hours and allowed to pass through an 83M sieve, thereby obtaining carrier particles 2.

The carrier particles 2 conformed the criteria for water-soluble and porous defined in the present specification.

FIG. 2 shows an electron micrograph (at the magnification of 2000) of the carrier particles 2. As shown in FIG. 2, the carrier particles 2 were spherical.

The specific surface area of the carrier particles 2 measured by [Method of measuring BET specific surface area] described above was 144 m²/g.

The average pore diameter of the carrier particles 2 was 13.8nm, and the total pore volume thereof was 0.50 cm³/g.

### <Example 3>

### [Step of obtaining precursor particles]

400 g of water (water temperature of 70°C), 390 g of trehalose (TREHALOSE P, manufactured by Hayashibara Co., Ltd.), 180 g of dextrin (PINEDEX #3, manufactured by Matsutani Chemical Industry Co., Ltd.), and 30 g of anhydrous citric acid were mixed, thereby obtaining an aqueous solution 3 having a solid content concentration of 60%.

The DE of the dextrin used was 25.

An aqueous solution 3 obtained under the following conditions was sprayed with a spray dryer, thereby obtaining precursor particles 3.

### (Spray conditions)

Inlet temperature: 140°C
Outlet temperature: 98°C
Disk rotation speed: 10000 rpm
Supply rate of aqueous solution: 100 ml/min

### [Step of obtaining carrier particles]

20 ml of anhydrous ethanol was added to 5 g of the obtained precursor particles, and the mixture was shaken and mixed in a water bath at 60°C for 30 minutes to 1 hour. After completion of the shaking, the mixture was allowed to stand, and the supernatant was removed by decantation. 20 ml of anhydrous ethanol was added thereto again, and the mixture was shaken and decanted in the same manner as described above, and this operation was repeated 11 times in total from the beginning. After the final decantation, 20 ml of anhydrous ethanol was added, mixed lightly, allowed to stand, and decanted to remove the supernatant. The mixture was dried at 50°C and -0.08 MPa, thereby obtaining carrier particles 3.

The carrier particles 3 conformed the criteria for water-soluble and porous defined in the present specification.

The specific surface area of the carrier particles 3 measured by the [Method of measuring BET specific surface area] described above was 7.64 m²/g.

### <Example 4>

### [Step of obtaining precursor particles]

1000 g of water (water temperature of 70°C), 1500 g of trehalose (TREHALOSE P, manufactured by Hayashibara Co., Ltd.), 600 g of dextrin (GLUCIDEX IT 29, manufactured by ROQUETTE), and 233 g of anhydrous citric acid were mixed, thereby obtaining an aqueous solution 4 having a solid content concentration of 70%.

The DE of the dextrin used was 29.

An aqueous solution 4 obtained under the following conditions was sprayed with a spray dryer, thereby obtaining precursor particles 4.

### (Spray conditions)

Inlet temperature: 140°C
Outlet temperature: 99°C to 100°C
Disk rotation speed: 13000 rpm
Supply rate of aqueous solution: 100 ml/min

### [Step of obtaining carrier particles]

6 g of the obtained precursor particles were added to 120 ml of anhydrous ethanol, and the mixture was shaken and mixed in a water bath at 40°C for 10 minutes. After completion of the shaking, the mixture was allowed to stand for 5 minutes, and the supernatant was removed by decantation. 120 ml of anhydrous ethanol was added thereto again, and the mixture was shaken and decanted in the same manner as described above, and this operation was repeated 10 times in total from the beginning. After the final decantation, 120 ml of anhydrous ethanol was added thereto, mixed lightly, allowed to stand for 5 minutes, and decanted to remove the supernatant. The mixture was dried at 50°C and -0.08 MPa, thereby obtaining carrier particles 4.

The carrier particles 4 conformed the criteria for water-soluble and porous defined in the present specification.

The specific surface area of the carrier particles 4 measured by [Method of measuring BET specific surface area] described above was 67.29 m²/g.

### <Example 5>

Carrier particles 5 were obtained by the same method as in Example 4 except that the dextrin was changed to dextrin (GLUCIDEX IT 33, ROQUETTE).

The carrier particles 5 conformed the criteria for water-soluble and porous defined in the present specification.

The specific surface area of the carrier particles 5 measured by [Method of measuring BET specific surface area] described above was 82.17 m²/g.

Since the carrier particles of Examples 1 to 5 are porous and have a high specific surface area, the amount of the functional ingredient carried can be increased. In addition, since the porous carrier particles of Examples 1 to 5 are water-soluble, the porous carrier particles are completely dissolved in vivo. Therefore, both the carried functional ingredient and the carrier particles are completely dissolved in vivo, and the functional ingredient can be completely released.

## Claims

1. Porous carrier particles comprising:
a water-soluble polymer that has a helical structure including glucose as a unit,
wherein the porous carrier particles have a BET specific surface area of 1 m²/g or greater.

2. The porous carrier particles according to Claim 1,
wherein the water-soluble polymer is a water-soluble polysaccharide.

3. The porous carrier particles according to Claim 2,
wherein the water-soluble polysaccharide is one or more selected from the group consisting of dextrin, dextran, agarose, and pullulan.

4. The porous carrier particles according to Claim 1 or 2,
wherein the porous carrier particles are spherical.

5. Particles carrying functional ingredient,
wherein a functional ingredient is retained inside the porous carrier particles according to Claim 1 or 2.

6. The particles carrying functional ingredient according to Claim 5,
wherein the functional ingredient is an active pharmaceutical ingredient.

7. A method of producing porous carrier particles having a BET specific surface area of 1 m²/g or greater, the method comprising:
a step of spray-drying a solution that contains a water-soluble polymer having a helical structure including glucose as a unit to obtain precursor particles; and
a step of mixing an organic solvent with the precursor particles to obtain a mixture and removing a liquid from the mixture.
